# EUROPÄISCHE PATENTSCHRIFT

(11) **EP 1 009 306 B1**
(45) Veröffentlichungstag und Bekanntmachung des Hinweises auf die Patenterteilung: **07.04.2004**
(21) Anmeldenummer: 98952564.7
(22) Anmeldetag: 04.09.1998
(51) Int. Cl.: A61B 18/12

(54) **ELEKTRODENANORDNUNG ZUR ELEKTROTHERMISCHEN BEHANDLUNG DES MENSCHLICHEN ODER TIERISCHEN KÖRPERS**
ELECTRODE ARRANGEMENT FOR ELECTROTHERMAL TREATMENT OF HUMAN OR ANIMAL BODIES
SYSTEME D'ELECTRODES POUR LE TRAITEMENT ELECTROTHERMIQUE DU CORPS HUMAIN OU DU CORPS ANIMAL

(30) Priorität: 04.09.1997 DE 19739699
(43) Veröffentlichungstag der Anmeldung: 21.06.2000
(73) Patentinhaber: Celon AG medical instruments, 14513 Teltow (DE)
(72) Erfinder: MÜLLER, Gerhard, D-14129 Berlin (DE); DESINGER, Kai, D-10827 Berlin (DE); STEIN, Thomas, D-10787 Berlin (DE)
(74) Vertreter: Eisenführ, Speiser & Partner
(86) Internationale Anmeldenummer: PCT/DE1998/002695
(87) Internationale Veröffentlichungsnummer: WO 1999/011186

(56) Entgegenhaltungen:
- WO-A-96/18349
- WO-A-96/34571
- WO-A-97/06739
- US-A- 5 588 960

## Beschreibung

Die Erfindung betrifft eine Elektrodenanordnung zur elektrothermischen Behandlung des menschlichen oder tierischen Körpers, insbesondere zur Elektrokoagulation.

Die Anwendung hochfrequenter Wechselströme (speziell im Frequenzbereich von 300 kHz bis 2 MHz) zur Erzeugung hoher Temperaturen zur Gewebekoagulation als chirurgisches Verfahren ist seit langem bekannt. In der Praxis werden zur Einbringung des HF-Stromes in das Gewebe mono- oder bipolare Elektrodenanordnungen angewandt.

Bei den monopolaren Anordnungen wird eine Elektrode - auch als Neutralelektrode bezeichnet - als großflächige Patientenableitung ausgelegt und nicht zu weit entfernt von der Eingriffsstelle am Patienten fixiert und geerste bzw. mit Masse verbunden. Eine zweite, vom Operateur gehandhabte Elektrode - auch als Aktivelektrode bezeichnet - ist mit dem Wechselstromgenerator verbunden. Diese ist in ihrer Form an die jeweilige Anwendung, insbesondere an die Größe des zu behandelnden Gewebsbereiches derart angepaßt gewählt, daß sowohl die Operationsdauer als auch die thermische Belastung des betroffenen Organs bzw. Körperbereiches vertretbar sind.

Bei Anordnungen zur bipolaren HF-Chirurgie sind beide Elektroden mit dem HF-Generator verbunden und in miteinander vergleichbaren Abmessungen ausgeführt und werden vom Operateur in unmittelbarer Nähe der Eingriffsstelle plaziert und in der Regel auch beide aktiv geführt. Es sind auch bipolare Elektrodenanordnungen bekannt, bei denen beide Koagulationselektroden an einem Katheter angeordnet sind.

Aus WO 97/17009 ist eine bipolare Elektrodenanordnung mit einem Flüssigkeitskanal bekannt, über den Spülflüssigkeit in den Eingriffsbereich eingebracht werden kann.

Aus WO 96/34569 sowie den im internatioanlen Recherchenberiecht dazu genannten Dokumenten sind Systeme und Verfahren zur Ablation von Körpergewebe unter Einhaltung einer vorberechneten maximalen Gewebstemperatur bekannt, bei denen während der eigentlichen Gewebskoagulation eine Fluidkühlung oder thermoelektrische Kühlung vorgesehen ist. Diese bekannten Anordnungen sind zur Einführung in Körperhöhlen über natürliche Zugänge gedacht.

Der Erfindung liegt die Aufgabe zugrunde, eine Elektrodenanordnung der eingangs genannten Art zu schaffen, die eine leichte und schnelle interstitielle Gewebskoagulation auch in relativ großen Behandlungsbereichen ermöglicht.

Die Aufgabe wird durch die Merkmale des Anspruchs 1 gelöst.

Die Erfindung schließt die grundsätzliche Lehre ein, die Elektrodenanordnung mechanisch in einer das direkte Eindringen in Körpergewebe erleichternden Weise auszuführen und zugleich thermische Mittel zur Einstellung eines vorteilhaften Wirktemperaturprofils für die Einführungsphase vorzusehen.

Diese Gedanken beruhen auf der Tatsache, daß die bekannten, während der eigentlichen Elektrothermie gekühlten Anordnungen zwar viele Vorteile gebracht haben,sich aber nicht gut dazu eignen, unter Kanalbildung zur interstitiellen Anwendung direkt (invasiv) in Körpergewebe eingeführt zu werden.

Aus diesem Grunde wird in der klinischen Praxis vielfach zunächst mit einem gesonderten Inzisionsbesteck und in einem zusätzlichen Arbeitsschritt des Operateurs ein Kanal zum Behnadlungsgebiet eröffnet, bevor der Elektrothermie-Applikator in diesem vorgeschoben wird.

Sie beruht weiter auf der Erkenntnis der Erfinder, daß ein "kalter" Applikator sich schwerer als ein etwas erwärmter einführen läßt.

Als vorteilhaft hat sich für die Einführungsphase eine kurzzeitige Temperierung oberhalb von ca. 30°C, speziell etwas oberhalb der Körpertemperatur, erwiesen. Sobald der Applikator den Behandlungsort erreicht hat und die eigentliche elektrothermische Behandlung eingeleitet wird, wird zur Einstellung eines auf optimalen Koagulationsablauf hin optimierten resultierenden Wirktemperaturprofils übergegangen. Auch in Zeitabschnitten dieser Phase kann eine Heizung zusätzlich zur Wärmeerzeugung über die Elektroden zweckmäßig sein.

In einer besonders wirkungsvollen und zugleich kostengünstigen Ausführung sind die Elektrode(n) bzw. der Elektrodenträger mit einem gegen den Körper abgeschlossenen Hohlraum versehen, der mit einer innerhalb eines vorbestimmten bereiches temperierbaren Fluidquelle verbunden ist, so daßdas geeignet temperierte Fluid die Elektrode bzw. deren Träger durchströmt. Als Fluid wird im Hinblick auf die geringen Kosten und die einfache und sichere Handhabung bevorzugt destilliertes Wasser eingesetzt. Daneben sind für spezielle Anwendungsfälle - ggfs. unter Beachtung spezifischer Sicherheitsvorkehrungen - auch andere als Wärmeübertragungsmittel bewährte Fluide einsetzbar, etwa Druckluft; Kohlendioxid oder Silikonöl.

In einer anderen möglichen Ausführung weist die Elektrode bzw. der Elektrodenträger eine thermoelektrische Heiz- und Kühleinrichtung auf, die etwa als Kombination aus Widerstandsheizern und Peltierelementen realisiert sein kann.

In einer leicht herzustellenden und zu handhabenden Ausführung ist der Elektrodenträger ein rohrförmiges, insbesondere zylindrisches Element aus elektrisch isolierendem Material, auf dessen Mantelfläche eine oder mehrere Elektrode(n) und in dessen Innerem die Temperiereinrichtung angeordnet sind. Zur Erleichterung des Eindringens in das Gewebe hat der Elektrodenträger zweckmäßigerweise ein sich verjüngendes bis annähernd kegelförmig spitzes distales Ende, und die Elektroden sind weitgehend bündig in die Mantelfläche des Trägers eingepaßt.

In der bevorzugten Ausführung als bipolare Anordnung umfaßt die Anordnung zwei an ein und demselben Elektrodenträger angebrachte Elektroden, insbesondere in axialer Reihung. Hierbei ist für beide Elektroden eine gemeinsame Temperiereinrichtung vorgesehen - beispielsweise die oben erwähnte Innenrohr-Gegenstromtemperierung.

In der bevorzugten Ausführungsform dieser Variante weist das Trägerelement einen zylindrischen Querschnitt auf, wobei die beiden Elektroden hohlzylindrisch ausgeführt und koaxial zur Längsachse des Trägerelements angeordnet sind. Die Elektroden können hierzu beispielsweise als metallische Beschichtung auf die Oberfläche des Trägerelements aufgebracht werden oder jeweils aus einer metallischen Hülse (z.B. aus Titan oder Nitinol) bestehen, die auf das Trägerelement aufgeschoben oder besser bündig eingefügt wird und mit diesem eine Preßpassung bildet.

In einer besonders einfachen und handhabungssicheren Ausführungsform dieser Anordnung wird die axiale Fixierung der Elektroden nicht durch ein durchgehendes Trägerelement bewirkt, sondern durch ein hohles Verbindungselement, welches die (ebenfalls hohlen) Elektroden an ihren Stirnseiten miteinander verbindet. Neben der axialen Fixierung der Elektroden hat das Verbindungselement auch die Aufgabe, die beiden Elektroden gegeneinander zu isolieren und besteht deshalb aus einem elektrisch isolierenden Material, bevorzugt PEEK (Polyetheretherketon). Die Elektroden, das Verbindungsstück und die Zuleitung für das Kühlmedium (etwa ein relativ steifer PTFE-Schlauch, der zugleich als Griffstück dient) sind hierbei vorzugsweise ring- bzw. rohrförmig und weisen denselben Querschnitt auf, so daß die Oberfläche des Katheters geschlossen zylindrisch ist, wodurch die Einführung in den Körper erleichtert wird und zudem unerwünschte Stromdichtespitzen weitgehend vermieden werden können.

In einer besonders variabel einsetzbaren, konstruktiv aber aufwendigeren Variante der bevorzugten bipolaren Anordnung ist der axiale Abstand zwischen den beiden Elektroden einstellbar, um die Stromdichte- und damit die Heizleistungsverteilung zusätzlich variieren zu können. Beträgt die Isolatorlänge zwischen den beiden Elektroden in axialer Richtung beispielsweise weniger als den doppelten Elektrodendurchmesser, so lassen sich vorteilhaft kugelförmige Koagulationsnekrosen erzielen, wohingegen die Form der Koagulationsnekrosen bei größeren Isolatorlängen eher oval ist.

Die geometrische Ausformung der Gewebekoagulation läßt sich durch die Temperierung - speziell durch die Wahl einer Heizung/Kühlung in bestimmter zeitlicher Abfolge- wesentlich beeinflussen.

Eine von der Erfindung Gebrauch machende Elektrochirurgievorrichtung schließt in bevorzugter Ausgestaltung neben der/den Elektrode(n) und der eigentlichen Kühleinrichtung Steuermittel zur Festlegung eines vorteilhaften Wirktemperaturprofils im Behandlungsbereich ein. Diese umfassen insbesondere eine über eine Steuersignalverbindung zur Zuführung eines Heiz- und/oder Kühlleistungs-Steuersignals mit der Kühleinrichtung verbundene Wirktemperaturprofil-Steuereinrichtung zur Steuerung der Heiz- bzw. Kühlleistung und/oder von deren räumlicher Verteilung.

Die Wirktemperaturprofil-Steuereinrichtung kann zudem zur Erzeugung und Zuführung eines Heizleistungs-Steuersignals zur Steuerung der Wechselstromleistung und/oder von deren räumlicher Verteilung ausgebildet und über einen Steuereingang mit der Wechselstromquelle verbunden sein, so daß sie neben der Kühleinrichtung auch die - als "Heizeinrichtung" im Gewebe wirkende - HF-Quelle steuern kann. Dies ermöglicht in besonders flexibler Weise eine Steuerung des Behandlungsregimes bei längerdauernden Eingriffen.

Die Wirktemperaturprofil-Steuereinrichtung umfaßt bevorzugt eine interaktiv programmierbare Berechnungseinheit zur Bestimmung simulierter zeitabhängiger Wirktemperaturprofile aufgrund von Parametern des Gewebes und der Elektrodenanordnung und von angenommenen Parametern der Wechselstromquelle und der Heiz- bzw. Kühleinrichtung und zur Variation der angenommenen Parameter zur Ermittlung eines optimierten Wirktemperaturprofils. In der Praxis wird man einen PC einsetzen, mit dem eine Bestimmung der räumlichen Temperaturverteilung und wahlweise von deren Zeitabhängigkeit erfolgen und auf dessen Bildschirm die simulierten Wirktemperaturprofile bildlich dargestellt werden können. Dem Operateur wird dadurch die Auswahl einer sinnvollen Kombination von Steuergrößen der Temperiereinrichtung und der HF-Quelle schon vor einem Eingriff erheblich erleichtert.

Durch Plazierung mindestens eines mit einem Eingang der Wirktemperaturprofil-Steuereinrichtung verbundenen trägheitsarm ansprechenden Temperaturfühlers in vorbestimmter Position relativ zur Elektrodenanordnung im Körper, insbesondere an einer Elektrode oder dem Elektrodenträger läßt sich eine Verifizierung bzw. Reskalierung eines simulierten Wirktemperaturprofils während des Eingriffs vornehmen. Es können daher zu jedem Zeitpunkt die im weiteren Behandlungsverlauf anzuwendenden Parameter neu justiert werden.

Zusätzliche Möglichkeiten hierfür erschließt das Vorsehen einer Einrichtung zur (speziell zeitabhängigen) Ermittlung der durch die Temperiereinrichtung erzeugten Heiz- bzw. Kühlleistung oder einer diese beeinflussenden Größe sowie einer Einrichtung zur Ermittlung der durch die Wechselstromquelle abgegebenen Wechselstromleistung oder einer diese beeinflussenden Größe.

Indem die Wirktemperaturprofil-Steuereinrichtung bevorzugt Mittel zur Bestimmung und Speicherung einer Zeitabhängigkeit des Kühlleistungs-Steuersignals und/oder des Heizleistungs-Steuersignals und zur Ausgabe des jeweiligen Steuersignals entsprechend der gespeicherten Zeitabhängigkeit aufweist, kann eine hinsichtlich der Handhabungen des Operateurs vorab festgelegte Behandlung hinsichtlich der Steuergrößen weitgehend automatisch ablaufen. Aktuelle Änderungen der Steuergrößen bleiben dabei selbstverständlich möglich. so daß der Operateur auch auf unvorhergesehene Ereignisse flexibel reagieren kann. Da derartige Änderungen erfaßt werden und in eine aktualisierte Simulationsrechung einfließen können, können dem Arzt zeitnah wiederum die Konsequenzen für den weiteren Ablauf des Eingriffs verdeutlicht werden.

Vorteilhafte Weiterbildungen der Erfindung sind im übrigen in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 eine grafische Darstellung einer im Ergebnis einer Simulationsrechnung erhaltenen Stromdichteverteilung längs einer bipolaren Elektrodenanordnung mit zwei axial gegeneinander versetzten Ringelektroden in Körpergewebe,
Figur 2 eine schematische Darstellung der sich um eine Elektrodenanordnung gemäß Fig. 1 ohne Temperierung ausbildenden Gewebszustände,
Figur 3 eine schematische Darstellung der sich um eine Elektrodenanordnung gemäß Fig. 1 mit temperierten Elektroden ausbildenden Gewebszustände,
Figur 4 eine vergleichende grafische Darstellung des Temperaturverlaufes im Bereich einer untemperierten und einer temperierten Elektrodenanordnung in Abhängigkeit vom Abstand von der Elektrodenoberfläche,
die Figuren 5a und 5b vergleichende Darstellungen der Zeitabhängigkeit der im Verlaufe einer Koagulationsbehandlung gemessenen Gewebeimpedanz und der an das Gewebe abgegebenen elektrischen Leistung bei einer untemperierten und einer temperierten Elektrodenanordnung,
die Figuren 6a und 6b schematische Längsschnittdarstellungen einer bipolaren Elektrodenanordnung mit zwei axial gegeneinander versetzten, feststehenden Ringelektroden und Fluid-Innentemperierung gemäß einer Ausführungsform der Erfindung,
Figur 7 eine schematische Darstellung einer bipolaren Elektrodenanordnung mit gegeneinaner verschieblichen Elektroden und Innentemperierung gemäß einer weiteren Ausführungsform der Erfindung,
Figur 8 eine Prinzipskizze einer Elektrochirurgievorrichtung mit Meßanordnung zur Bestimmung wesentlicher Behandlungsgrößen,
Figur 9 ein Struktogramm eines Simulationsprogramms zur On-line-Dosimetrie bei der interstitiellen HF-Thermotherapie,
Figur 10 eine bildliche Darstellung eines Simulationsergebnisses und
Figur 11 ein Blockschaltbild der Wirktemperaturprofil-Steuereinrichtung der Elektrochirurgievorrichtung gemäß Fig 8.

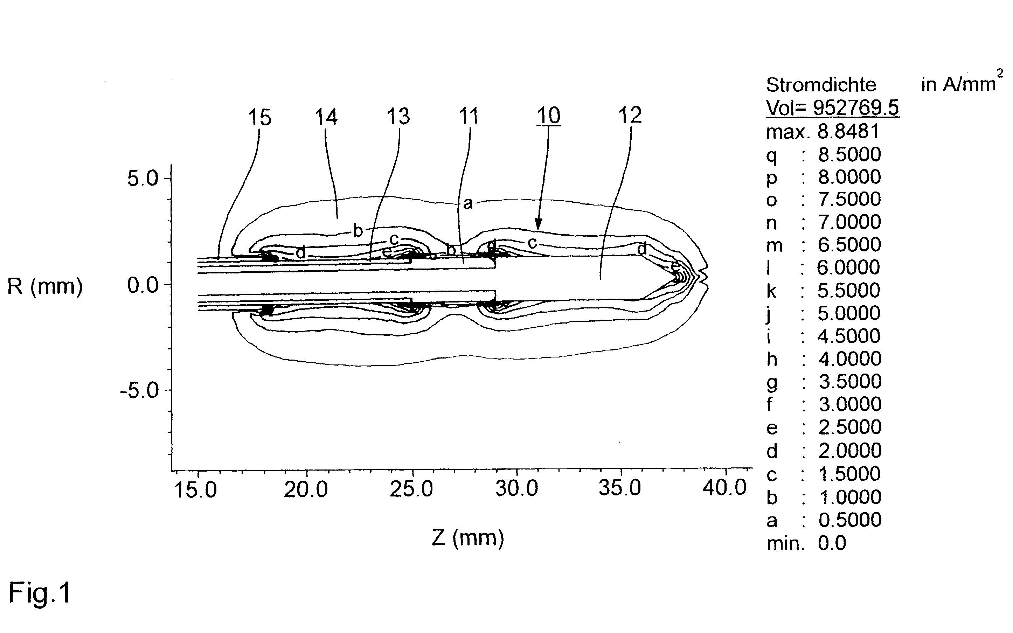

Fig. 1 zeigt in einer schematischen Längsschnittdarstellung eine im Ergebnis einer Simulationsrechnung der Erfinder erhaltene Stromdichteverteilung längs einer bipolaren Elektrodenanordnung 10 mit einer Spitzenelektrode 12 und einer durch einen isolierenden Elektrodenträger 11 axial mit einem Abstand zur Spitzenelektrode 12 fixierten und gegenüber dieser isolierten Ringelektrode 13 in Körpergewebe 14. Der Abschnitt der Elektrodenanordnung 10 proximal der Ringelektrode 13 ist mit einer isolierenden Hülle 15 umgeben. Neben der Längsschnittdarstellung sind tabellarisch die bei der Darstellung des Simulationsergebnisses zugrundegelegten Stromdichtestufen a bis q und die maximale Stromdichte (max) aufgelistet.

Es ist bereits an den wenigen im Längsschnitt überhaupt erkennbaren Stromdichteflächen aus der Tabelle (im wesentlichen nur den durch die Linien a bis g repräsentierten Flächen) zu erkennen, daß sich an der Spitze und jeweils in den Grenzbereichen der Elektroden 12, 13 zu den isolierenden Abschnitten 11, 15 der Anordnung Stromdichtespitzen ausbilden und die Stromdichte insgesamt schon mit geringem Abstand von den Oberflächen stark absinkt. Dies zeigt, daß davon auszugehen ist, daß der weitaus größte Teil der durch die Elektrodenanordnung 10 in das Körpergewebe 14 eingetragenen elektrischen Energie in den unmittelbar an die Elektrodenoberflächen angrenzenden Gewebsbereichen in thermische Energie umgesetzt wird. Die Gewebekoagulation beginnt in den Zonen höchster Stromdichte an den einander zugewandten Elektrodenkanten.

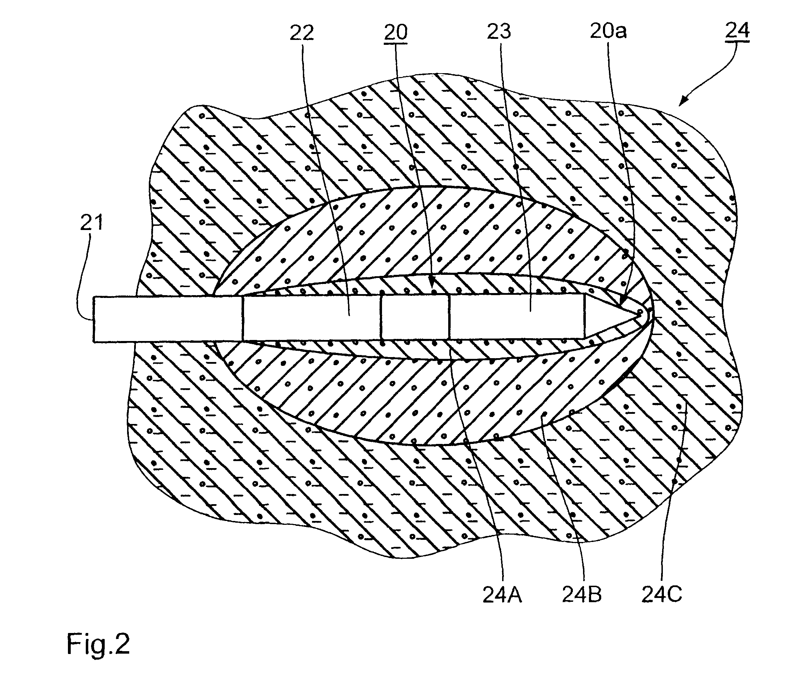

Dies führt, wie in Fig. 2 skizziert ist, zur Ausbildung einer Austrockungszone annähernd in der Gestalt eines schlanken Roatationsellipsoids um eine derartige Elektrodenanordnung herum. Die in Fig. 2 dargestellte Elektrodenanordnung 20 umfaßt - in geringfügiger Modifikation gegenüber der in Fig. 1 gezeigten Ausbildung - zwei in einen zylindrischen Träger 21 eingelassene, gleichlange zylindrische Elektroden 22, 23, während die Spitze 20a hier isolierend ausgeführt ist. Das umgebende Gewebe 24 ist in Abhängigkeit von der Temperatur und den dadurch bewirkten Gewebsveränderungen in die Austrocknungszone 24A, eine Koagulationszone 24B und einen strukturell unveränderten ("nativen") äußeren Bereich 24C unterteilt. Direkt an der Oberfläche der Anordnung kann zudem - insbesondere bei hohem Leistungseintrag - eine (in der Figur nicht dargestellte) Karbonisierungsschicht bzw. - zone ausgebildet sein.

Die Ausbildung der Austrocknungszone 24A verschlechtert im Hinblick auf eine elektrochirurgische Behandlung dramatisch die elektrischen Eigenschaften des Behandlungsbereiches. Die Erhöhung der Impedanz infolge des Verschwindens von Gewebsflüssigkeit führt zu einer erheblichen Verringerung der in diese Zone - und damit insgesamt in das Gewebe - einkoppelbaren elektrischen Energie. Zudem stellt eine Karbonisierungszone einen Bereich niedriger thermischer Leitfähigkeit dar und verschlechtert die Behandlungseffizienz zusätzlich.

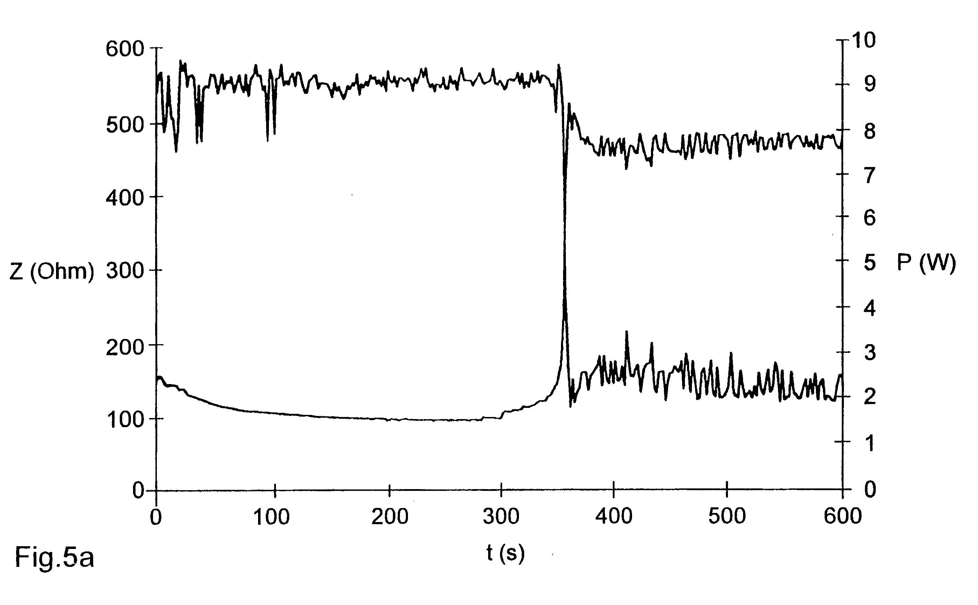

Dies wurde in Messungen der Erfinder bestätigt, wie Fig. 5a zeigt. Diese Figur ist eine Darstellung der Zeitabhängigkeit der im Verlaufe einer Koagulationsbehandlung gemessenen Gewebeimpedanz und der an das Gewebe abgegebenen elektrischen Leistung bei einer untemperierten Elektrodenanordnung. In der Figur ist zu erkennen, daß die Impedanz nach einem anfänglichen Absinken, das auf eine Anlagerung von Gewebsflüssigkeit an die Elektrodenoberfläche zurückzuführen sein dürfte, über eine gewisse Behandlungsdauer nahezu konstant bei einem Wert um 100 Q liegt. Dies ermöglicht (bei der hier auf eine Maximalleistung von 10 W eingestellten Anordnung) einen Energieeintrag von ca. 9 W. Nach ca. 6 min Behandlungsdauer steigt die Impedanz aber infolge der Austrocknung der Elektrode-Gewebe-Grenzschicht sprunghaft auf etwa das Vierfache an, was eine Verringerung der umgesetzten elektrischen Leistung auf 2-3 W zur Folge hat.

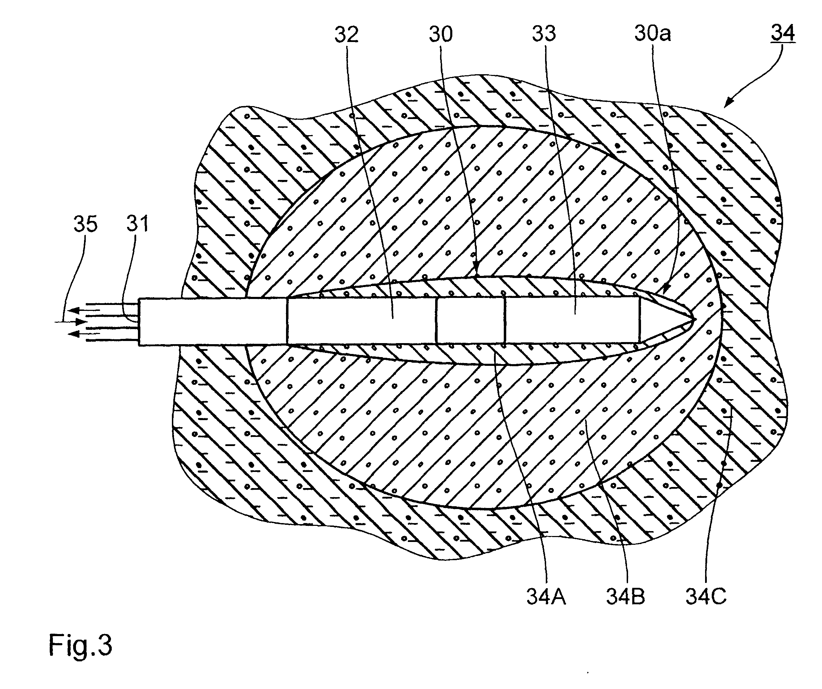

In Fig. 3 ist schematisch dargestellt, wie sich das Vorsehen einer Innentemperierung der Elektrodenanordnung auf die sich im Behandlungsbereich ausbildenden Gewebszustände auswirkt. Die in Fig. 3 skizzierte Elektrodenanordnung 30 umfaßt - insoweit übereinstimmend mit der in Fig. 2 gezeigten Ausbildung - zwei in einen zylindrischen Träger 31 eingelassene, gleichlange zylindrische Elektroden 32, 33 und eine isolierende Spitze 30a. Mit Ziffer 35 und den Pfeilen am proximalen Ende des dargestellten Teils der Anordnung ist schematisch eine Fluid-Gegenstromtemperierung bezeichnet. Das umgebende Gewebe 34 ist in eine relativ kühle Zone 34A, eine Koagulationszone 34B und einen strukturell unveränderten äußeren Bereich 34C unterteilt. Die Ausbildung einer Karbonisierungsschicht wird mit einer solchen Anordnung entweder überhaupt nicht beobachtet, oder diese beginnt jedenfalls nicht direkt an der Elektrodenoberfläche.

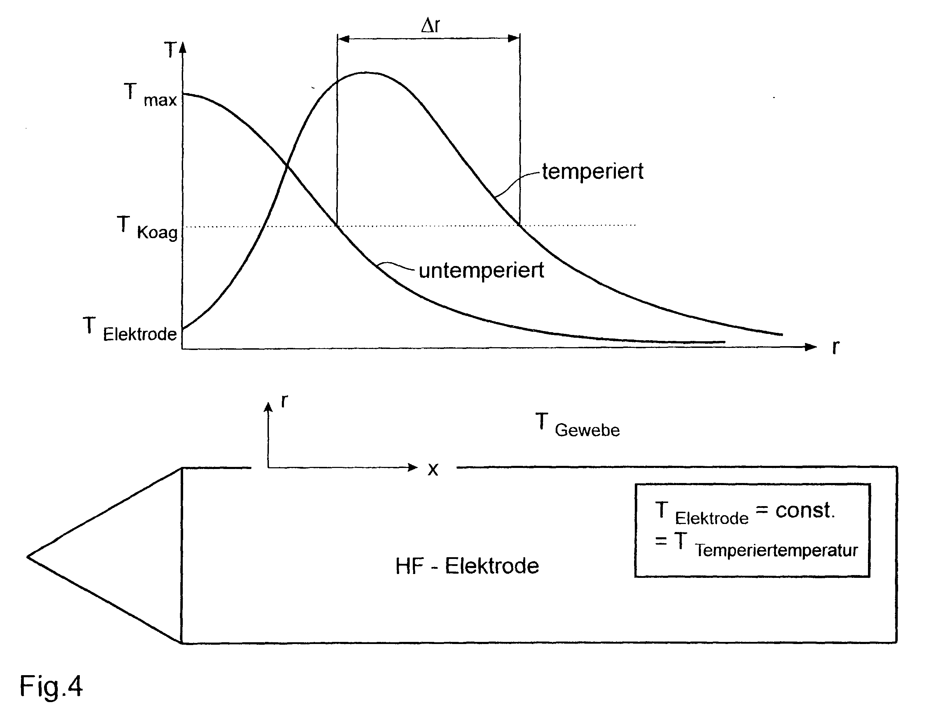

Die in Fig. 3 skizzierten Verhältnisse lassen sich auch einer vergleichenden grafischen Darstellung des räumlichen Temperaturverlaufes im Bereich einer untemperierten und einer temperierten Elektrodenanordnung entnehmen, wie sie im oberen Bereich von Fig. 4 gegeben ist. Der untere Teil dieser Figur stellt eine Skizze zur Erläuterung der in den Funktionskurven verknüpften Größen und zugleich der äußeren Form der Elektrodenanordnung dar.

Aus den Funktionskurven T(r) - die die Verhältnisse zu einem Zeitpunkt kurz nach Beginn des Anlegens der Wechselspannung zeigen - ist deutlich zu ersehen, daß sich die Koagulationszone bei einer temperierten Anordnung um einen Radius-Differenzwert Δr ausweitet. Schon dies belegt die höhere Effizienz der gekühlten Anordnung. Zur Funktionskurve für die temperierte Anordnung ist zu bemerken, daß - je nach Einstellung der Temperatur des Temperierungsfluids - in der Praxis nach einer gewissen Behandlungsdauer auch im Elektroden-Grenzbereich die Koagulationstemperatur überschritten wird, so daß auch in diesem Bereich ein Behandlungserfolg eintritt.

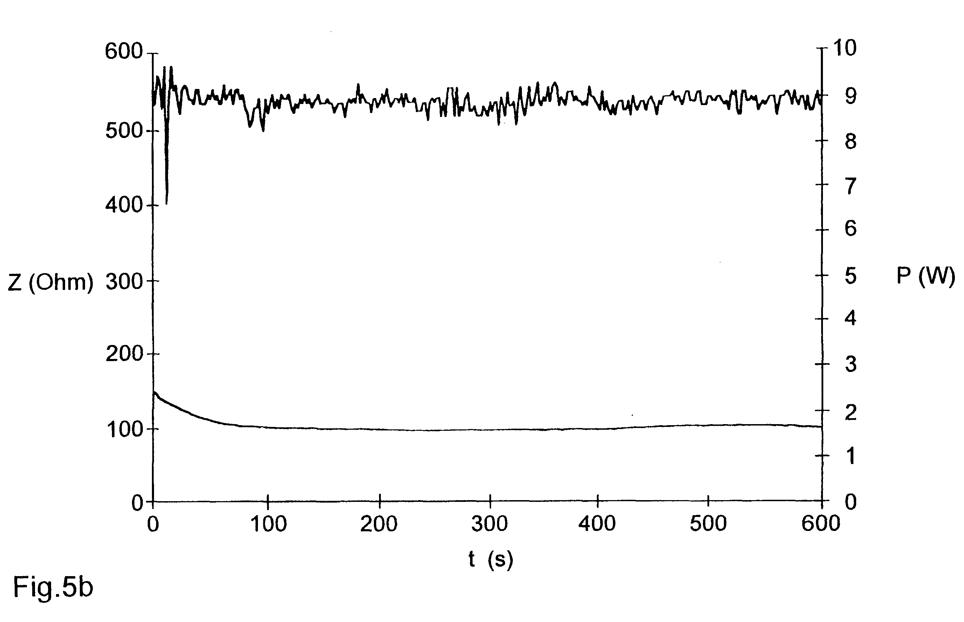

Fig. 5b zeigt die Zeitabhängigkeit der Gewebsimpedanz und der an das Gewebe abgegebenen elektrischen Leistung bei einer temperierten Elektrodenanordnung. Es ist zu erkennen, daß die Impedanz (wiederum nach anfänglichem Absinken) und die umgesetzte Leistung über die Behandlungsdauer praktisch konstant bleiben.

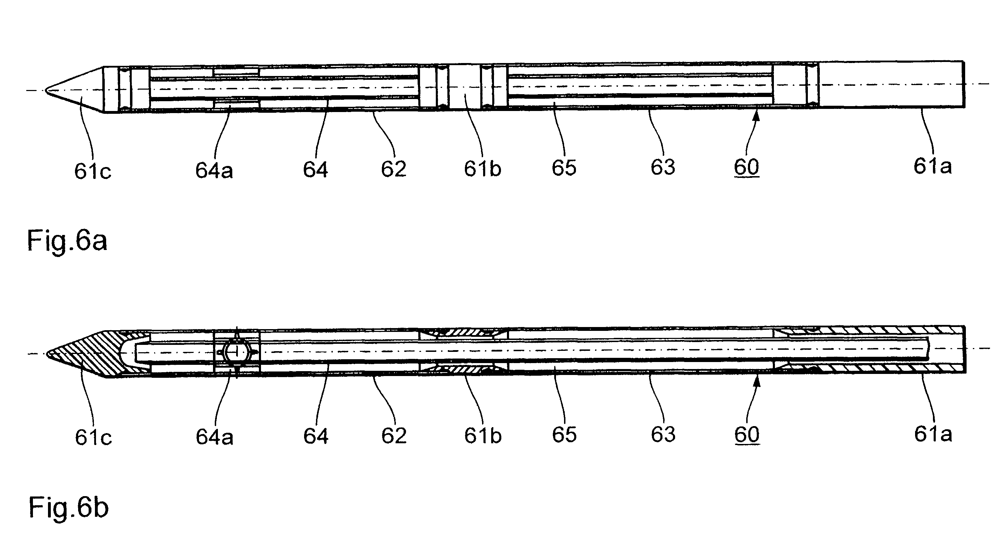

Fig. 6a und 6b sind eine teilweise geschnittene sowie eine Längsschnittdarstellung (in verschiedenen Schnittebenen) einer temperierten bipolaren Elektrodenanordnung 60 als Applikator für die HF-induzierte interstitielle Thermotherapie von pathologischem Gewebe. Diese umfaßt einen rohrförmigen Kunststoffträger 61a, zwei axial gegeneinander versetzte, feststehende metallische Ringelektroden 62, 63, die über einen ringförmigen Kunststoff-Zwischenträger 61b fixiert und gegeneinander isoliert sind, eine Kunststoffspitze 61c, ein Innenrohr 64 sowie einen Innenrohr-Abstandshalter 64a. Die Elektroden sind über (in der Figur nicht dargestellte) Leitungen aus hochflexibler Kupferlitze an einen HF-Generator angeschlossen.

Die Elektroden 62, 63 haben - je nach Anwendungsgebiet - einen Durchmesser im Bereich zwischen 1 und 5 mm und eine Länge von 2 bis 30 mm. Sie sind im Beispiel als NITINOL-Röhrchen ausgeführt und auf die aus hochtemperaturfestem PEEK (Polyethyletherketon) gefertigten Kunststoffteile 61a, 61b und 61c aufgeschoben und mittels hochtemperaturfestem Kleber aufgeklebt. Der Durchmesser der Kunststoffteile ist an den der Elektroden angepaßt; die Länge des Zwischenstücks beträgt das 1,5- bis 3-fache des Elektrodendurchmessers. Die auf den Kunststoffteilen aufliegenden Elektrodenbereiche sind mit PTFE beschichtet. Das Innenrohr 64 besteht aus PTFE. Anstelle der genannten können auch andere bewährte biokompatible, in Körpergewebe leicht gleitfähige und ausreichend temperaturbeständige Materialien eingesetzt werden. Zur Gewährleistung einer leichten kanalbildenden Einführung direkt in Körpergewebe sind neben einer geeigneten Formgebung des Applikators der Einsatz polierter Elektroden und ggfs. eine Gleitbeschichtung von Vorteil.

Die Elektrodenanordnung 60 wird mit einer HF-Leistung von bis zu 100 W (bei der Behandlung von Lebermetastasen oder benigner Prostata-Hyperplasie beispielsweise 50-60 W) betrieben. Als Temperierungsmedium wird in das Innenrohr 64 steriles destilliertes Wasser mit einer zeitabhängig einstellbaren Temperatur im Bereich zwischen 0 und 80°C (beispielsweise beim Einführen und zur Beginn der Elektrothermie über 30°C und anschließend Raumtemperatur) unter einem Druck zwischen 0 und 3 bar (beispielsweise 1 bar) mit einer Flußrate (flow) bis zu 200 ml/min (im o.g. Beispiel von 40-60 ml/min) eingeleitet. Das Wasser durchströmt das Innenrohr bis zu dessen distalem Ende im Bereich der Spitze 61c, gelangt dort in den Ringraum 65 zwischen dem Innenrohr und der Außenwandung der Anordnung und strömt in diesem zurück nach außerhalb des Körpers, wo es abgeleitet wird.

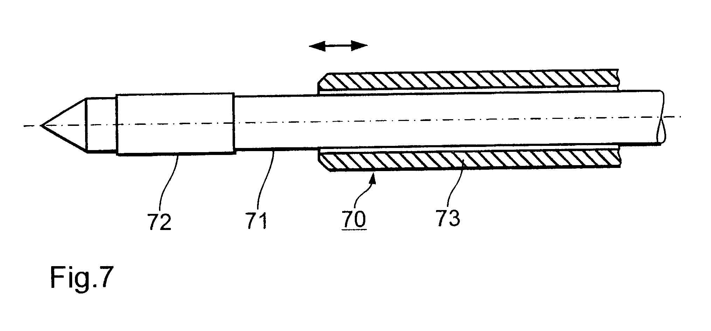

Figur 7 zeigt schließlich in schematischer Darstellung einen weiteren Elektrodenkatheter 70, der eine Einstellung des Elektrodenabstands ermöglicht, um die Feld- und Stromdichteverteilung im Therapiegebiet beeinflussen zu können. Hierzu weist der Katheter 70 ein zylindrisches Trägerelement 71 aus elektrisch isolierendem Material auf, nahe dessen distalem Ende eine erste Elektrode 72 als ringförmige metallische Beschichtung aufgebracht ist. Das Trägerelement 71 wird in seinem isolierenden Bereich von einer es umgebenden hohlzylindrisch ausgeführten zweiten Elektrode 73 axial verschieblich geführt, um den Elektrodenabstand einstellen zu können und damit eine zusätzliche Möglichkeit zur Variation des Wirktemperaturprofils im Behandlungsbereich verfügbar zu haben.

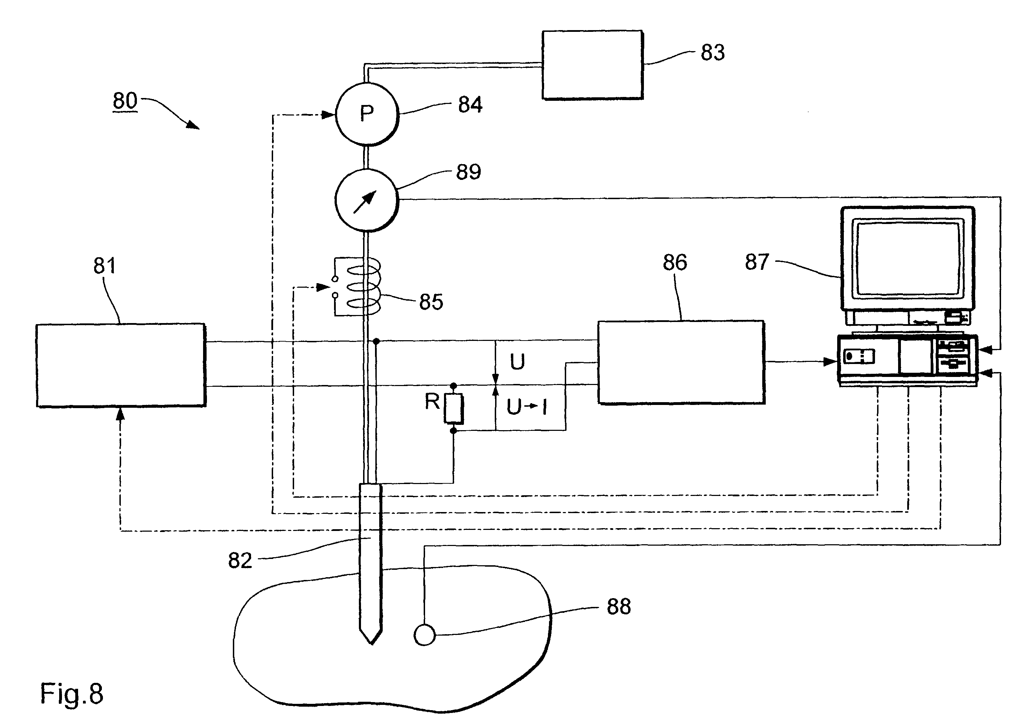

Fig. 8 ist eine Prinzipskizze einer Elektrochirurgievorrichtung 80 mit einem HF-Generator 81, einem Elektrodenkatheter 82 (auch als "HF-Nadel" bezeichnet), einem Temperierflüssigkeitsbehälter 83, einer Temperierflüssigkeitspumpe 84 und einem Temperierflüssigkeitsheizer 85 sowie einer Meß- und Auswertungsanordnung zur Festlegung wesentlicher Behandlungsgrößen. Die Meß- und Auswertungsanordnung umfaßt hier ein Speicheroszilloskop 86 zur Erfassung der elektrischen Größen, einen Durchflußmengenmesser 89 zur Erfassung des Temperierfluiddurchsatzes, einen im Behandlungsbereich angeordneten T-Sensor 88 und über eine geeignete Meßdaten-Schnittstelle mit den Meßgeräten 86, 88 und 89 verbundenen PC 87 zur Auswertung der Meßwerte.

Diese Anordnung ist durch die Auswahl geeigneter Stellglieder, d.h. eines steuerbaren HF-Generators 81, einer steuerbaren Fluidpumpe 84 und eines steuerbaren Fluidheizers 85, die durch - in der Figur strichpunktiert gezeichnete - Steuerleitungen mit dem PC 87 (oder einer gesonderten, mit dem PC verbundenen Steuereinheit) verbunden sind, zur Steuerung der wesentlichen Behandlungsgrößen HF-Ausgangsleistung und Heiz- bzw. Kühlleistung im Verlaufe eines elektrochirurgischen Eingriffs anhand einer Wirkleistungs- und Impedanz- und/oder Temperaturmessung im Behandlungsraum einsetzbar.

Insbesondere läßt sich für die Einführungsphase mittels des Fluidheizers 85 die Fluidtemperatur und damit (bei ausgeschaltetem HF-Generator 80) die Temperatur der HF-Nadel 81 auf einen Wert im Bereich der Körpertemperatur oder darüber erhöhen. Die Fluidpumpe 84 kann in dieser Phase mit relativ niedriger Förderleistung bzw. intermittierend betrieben werden, und auf eine T-Regelung kann ggfs. verzichtet werden. Nach Positionierung der HF-Nadel und bei Einschaltung des HF-Generators - oder noch günstiger mit einer vorbestimmten oder aus Signalen des T-Fühlers abgeleiteten Zeitverzögerung nach dessen Einschalten - wird der Fluidheizer ausgeschaltet. In dieser Phase wird im Normalfall die Fluidpumpe aufgrund des vorbestimmten Ziel-Temperaturfeldes und unter Auswertung der Signale des T-Fühlers gesteuert, so daß dann eine T-Regelung stattfindet. Es kann aber auch während des gesamten Eingriffs eine differenzierte Ansteuerung der Fluidpumpe und des Fluidheizers anhand eines vorbestimmten zeitabhängigen Ziel-Temperaturfeldes erfolgen, das die besonderen Erfordernisse der Einführungsphase berücksichtigt.

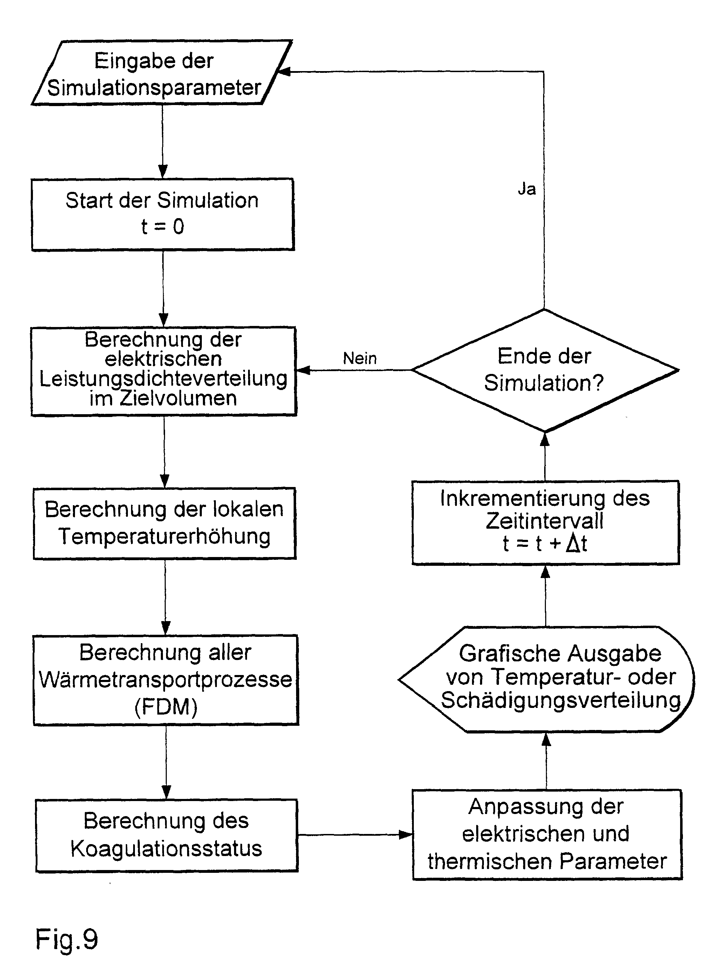

Methodische Grundlage einer Darstellung des Therapieverlaufes ist eine auf der Methode der finiten Differenzen zur Lösung der die elektrischen und thermischen Vorgänge beschreibenden Differentialgleichungen beruhende Simulationsrechnung. Deren grundsätzlicher Ablauf ist in Fig. 9 skizziert, die keines weiteren Kommentars bedarf.

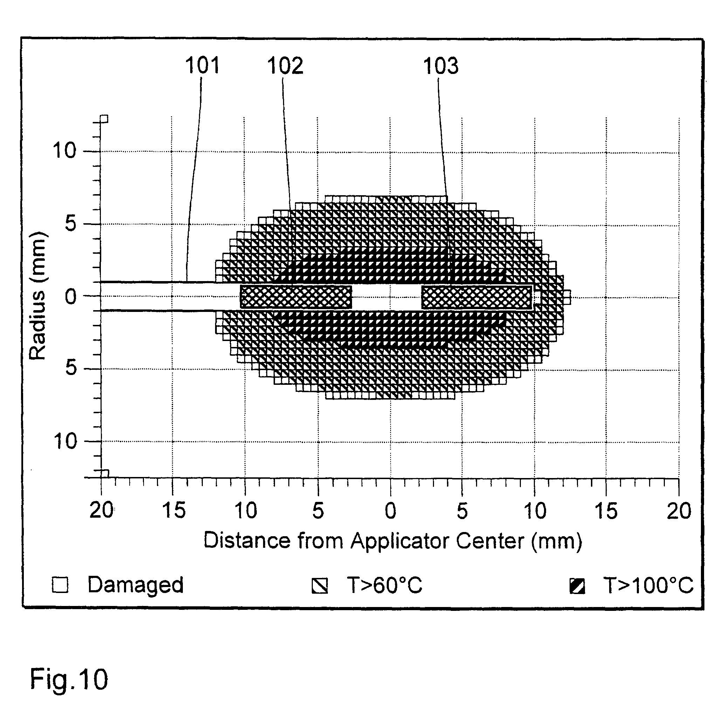

Fig. 10 gibt eine bildliche Darstellung eines Simulationsergebnisses, wie sie auf einem Computerbildschirm erscheint. Dargestellt sind die T-Verteilung und die Grenze des Behandlungsbereiches ("Damaged") um einen Applikator, der auf einem isolierenden Trägerkörper 101 zwei axial gereihte Elektroden 102 und 103 trägt.

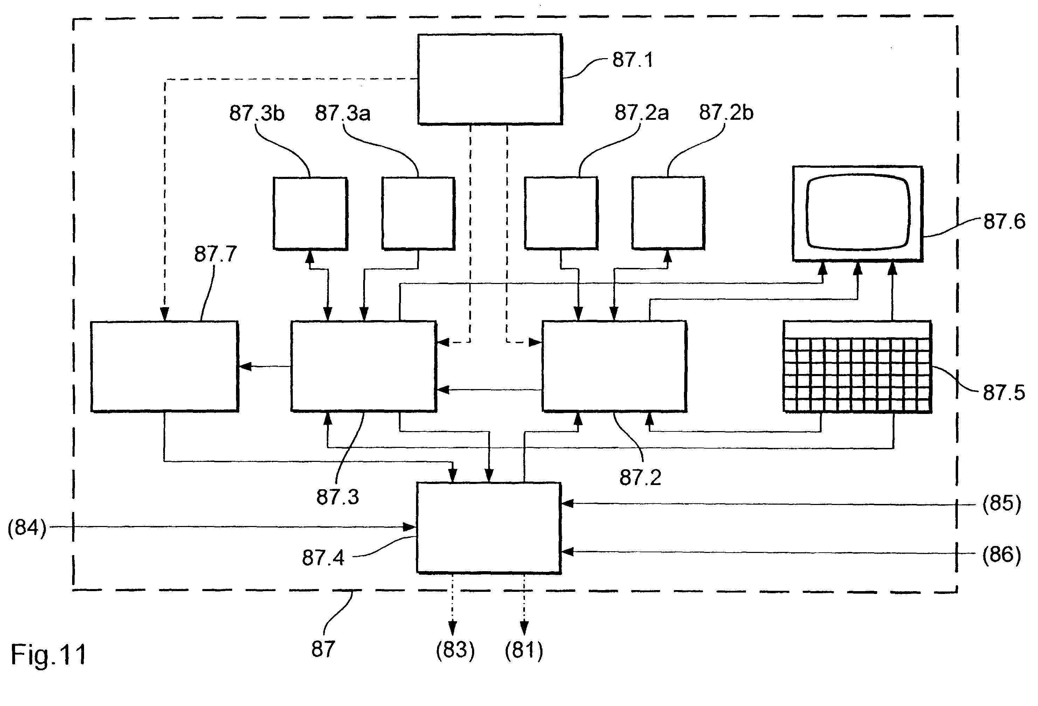

Fig. 11 zeigt ein Funktions-Blockschaltbild eines Ausführungsbeispiels der integrierten Auswertungs- und Steuereinrichtung 87 des HF-Applikationssystems 80 aus Fig 8. Die peripheren Komponenten sind in Fig. 8 dargestellt und daher in Fig. 11 weggelassen, und auch die obigen Erläuterungenn zu den grundsätzlichen Steuerfunktionen werden in Bezug auf Fig. 11 nicht wiederholt.

Die Auswertungs- und Steuereinrichtung 87 umfaßt als Hauptkomponenten eine Ablaufsteuerung (Controller) 87.1, eine Wirktemperaturprofil-Berechnungseinheit 87.2 und eine Steuergrößen-Berechnungseinheit 87.2. Diesen Komponenten sind jeweils in üblicher Weise gesonderte Programm- und Datenspeicher 87.2a, 87.2b bzw. 87.3a, 87.3b und gemeinsam eine I/O-Schnittstelle 87.4, eine Eingabeeinheit 87.5 und eine Anzeigeeinheit 87.6 zugeordnet. Der Steuergrößen-Berechnungseinheit 87.3 ist ausgangsseitig zusätzlich ein Steuerablaufspeicher 87.6 zur Speicherung von berechneten Zeitabhängigkeiten des Kühl- und des Heizleistungs-Steuersignals zugeordnet, dessen (nicht gesondert dargestellte) Zugriffssteuerung mit dem Controller 87.1 verbunden ist.

Im Programm- und im Datenspeicher 87.2a, 87.2b der Wirktemperaturprofil-Berechnungseinheit 87.2 sind insbesondere das oben erwähnte Simulationsprogramm ("Dosimetrieprogramm") und die zur Ausführung erforderlichen Datensätze gespeichert, die durch aktuelle Eingaben über die Eingabeeinheit 87.5 bedienerseitig und bei Eingang neuer Meßdaten über die Schnittstelle 87.4 automatisch aktualisiert werden können. Damit ist in jeder Phase einer Behandlung - einschließlich der kanalbildendne Einführung der HF-Nadel - die Gewinnung eines aktuellen Simulationsergebnisses für das Wirktemperaturprofil (vgl. Fig. 10) und damit einer Prognose über den weiteren Behandlungsverlauf möglich.

Im Programm- und im Datenspeicher 87.3a, 87.3b der Steuergrößen-Berechnungseinheit 87.3 sind insbesondere Algorithmen und Parametersätze gespeichert, die eine Zuordnung konkreter Steuerdatensätze zu Wirktemperaturprofildatensätzen ermöglichen, ggfs. auch bereits direkt zugreifbare Steuerdatentabellen. Komplette Steuerdatensätze für eine Gesamtbehandlung können nach Abschluß einer vorbereitenden oder aktualisierten Simulationsrechung in den Steuerablaufspeicher 87.6 verschoben werden, von wo sie über den Controller 87.1 zur automatischen Zeitablaufsteuerung der Behandlungsparameter zur Einstellung der Fluidpumpe 84, des Fluidheizers 85 und des HF-Generators 81 (Fig. 8) ausgegeben werden können. Auch hierbei ist ein korrigierender Eingriff des Operateurs in jeder Phase möglich.

Die Erfindung beschränkt sich in ihrer Ausführung nicht auf die vorstehend angegebenen bevorzugten Ausführungsbeispiele. Vielmehr ist eine Anzahl von Varianten denkbar, welche von der beanspruchten Lösung auch in anders gearteten Ausführungen Gebrauch macht.

## Patentansprüche

1. Elektrochirurgievorrichtung mit einem Elektrodenträger (31, 61a-c) mindestens einer Elektrode (32, 33, 62, 63) auf dem Elektrodenträger (31, 61a-c), einer mit der Elektrode (32, 33, 62, 63) über ein Kabel leitenden verbundenen Wechselstromquelle (81) und mit einer Temperiereinrichtung (35, 64, 65, 84) für die Elektrode (32, 33, 62, 63) und den Elektrodenträger (31, 61a-c), wobei der Elektrodenträger (31, 61a-c) an seinem distalen Ende zur direkten Einführung in Körpargewebe spitz ausgebildet ist, und wobei Mittel (85) vorgesehen sind, mit Hilfe derer die Elektrode (32, 33, 62, 63) und der Elektrodenträger (31, 61a-c) unabhängig von der elektrischen Stromstärke durch die Elektrode (32, 33, 62, 63) heizbar sind, die Elektrode (32, 33, 62, 63) und/oder der Elektrodenträger (31, 61a-c) einen Hohlraum (64, 65) aufweist und die Temperiereinrichtung (35, 64, 65, 84) eine zeitsteuerbare Heizeinrichtung zur Temperierung des Fluids einer Fluidquelle (83, 85) enthält, die über eine Durchflussmengensteuereinrichtung (84, 87) mit der Elektrode (32, 33, 62, 63) und/oder dem Elektrodenträger (31, 61a-c) in Verbindung steht und eine Wirktemperaturprofil-Steuereinrichtung (87) vorgesehen ist, die über eine Steuersignalverbindung mit der Temperiereinrichtung (35, 64, 65, 84) verbunden ist.

2. Elektrochirurgievorrichtung nach Anspruch 1, **dadurch gekennzeichnet, daß** Elektrode (32, 33, 62, 63) und Elektrodenträger (31, 61a-c) auf eine Temperatur von mehr als 30 Grad heizbar sind.

3. Elektrochirurgievorrichtung nach Anspruch 2, **dadurch gekennzeichnet, daß** Elektrode (32, 33, 62, 63) und Elektrodenträger (31, 61a-c) auf eine Temperatur von mehr als 37 Grad heizbar sind.

4. Elektrochirurgievorrichtung nach einem der Ansprüche 1 bis 3, **dadurch gekennzeichnet, daß** die Elektrode (32, 33, 62, 63) und/oder der Elektrodenträger (31, 61a-c) eine thermoelektrische Heiz- und Kühleinrichtung aufweist.

5. Elektrochirurgievorrichtung nach einem der vorstehenden Ansprüche, **dadurch gekennzeichnet, daß** die Wirktemperaturprofil-Steuereinrichtung (87) zur Steuerung der Wechselstromleistung über einen Steuereingang mit der Wechselstromquelle (81) verbunden ist.

6. Elektrochirurgievorrichtung nach Anspruch 5, **dadurch gekennzeichnet, daß** die Wirktemperaturprofil-Steuereinrichtung (87) eine interaktiv programmierbare Berechnungseinheit (87.2) zur Bestimmung simulierter, zeitabhängiger Wirktemperaturprofile aufgrund von Parametern des Gewebes und der Elektrode (82) und von angenommenen Paramtern der Wechselstromquelle (81) und der Temperiereinrichtung (83), und zur Variation der angenommenen Parameter zur Ermittlung eines optimierten, zeitabhängigen Wirktemperaturprofils umfaßt.

7. Elektrochirurgievorrichtung nach einem der Ansprüche 5 oder 6, **gekennzeichnet durch** mindestens einen trägheitsarmen Temperaturfühler (86), der mit einem Eingang der Wirktemperaturprofil-Steuereinrichtung (87) verbunden ist und der im Körper benachbart zur Elektrode (32, 33, 62, 63) oder dem Elektrodenträger (31, 61 a-c) angeordnet werden kann.

8. Elektrochirurgievorrichtung nach Anspruch 7, **dadurch gekennzeichnet, daß** der Temperaturfühler (86) mit einem Eingang der Berechnungseinheit (87.2) verbunden ist und daß die Berechnungseinheit (87.2) Mittel zur Verifizierung oder Korrektur eines simulierten, zeitabhängigen Wirktemperaturprofils aufgrund des Meßsignals des Temperaturfühlers aufweist.

9. Elektrochirurgievorrichtung nach einem der Ansprüche 5 oder 6, **gekennzeichnet durch** Impedanzmessmittel (86), die zum Messen der Impedanz zwischen zwei Elektroden (32, 33; 62, 63) auf dem Elektrodenträger (31;61a, 61b) ausgebildet und mit einem Eingang der Wirktemperaturprofil-Steuereinrichtung (87) verbunden sind.

10. Elektrochirurgievorrichtung nach Anspruch 9, **dadurch gekennzeichnet, daß** die Impedanzmessmittel (86) zur Steuerung einer über die Elektroden (32, 33; 62, 63) abzugebenden HF-Ausgangsleistung mit der Wechselstromquelle (81) verbunden sind.

11. Elektrochirurgievorrichtung nach Anspruch 9, **dadurch gekennzeichnet, daß** die Wirktemperaturprofil-Steuereinrichtung (87) zur Steuerung der Heizund Kühlleistung durch eine Fluidpumpe (84) und/oder einen Fluidheizer (85) in Abhängigkeit einer Impedanzmessung durch die Impedanzmessmittel (86) ausgebildet ist.

12. Elektrochirurgievorrichtung nach einem der vorstehenden Ansprüche, **dadurch gekennzeichnet, daß** die Wirktemperaturprofil-Steuereinrichtung Mittel (87, 1, 87.2, 87.6) zum Speichern und Abrufen der Zeitabhängigkeit von Steuersignalen und zur Ausgabe von Steuersignalen entsprechend einer gespeicherten Zeitabhängigkeit aufweist.

13. Elektrochirurgievorrichtung nach einem der vorstehenden Ansprüche, **dadurch gekennzeichnet, daß** der Elektrodenträger (31, 61a-c) ein rohrförmiges Element (61 a, 61 b) aus elektrisch isolierendem Material mit sich verjüngendem, insbesondere kegelförmigen distalen Ende aufweist, auf dessen Mantelfläche die Elektrode (62, 63) und in dessen Innerem die Temperiereinrichtung (64, 65) angeordnet sind.

14. Elektrochirurgievorrichtung nach einem der vorstehenden Ansprüche, **gekennzeichnet durch** zwei Elektroden (32, 33; 62, 63) auf dem Elektrodenträger (31; 61 a, 61 b).

15. Elektrochirurgievorrichtung nach einem der vorstehenden Ansprüche, **dadurch gekennzeichnet, daß** ein Temperierfluid steriles, destilliertes Wasser ist.

## Claims

1. Electrosurgery apparatus comprising an electrode carrier (31, 61 a-c) of at least one electrode (32, 33, 62, 63) on the electrode carrier (31, 61a-c), an alternating current source (81) conductively connected to the electrode (32, 33, 62, 63) by way of a cable, and a temperature control device (35, 64, 65, 84) for the electrode (32, 33, 62, 63) and the electrode carrier (31, 61a-c), wherein the electrode carrier (31, 61a-c) is of a pointed configuration at its distal end for direct insertion into body tissue, and wherein means (85) are provided, with the aid of which the electrode (32, 33, 62, 63) and the electrode carrier (31, 61a-c) can be heated independently of the electric current intensity flowing through the electrode (32, 33, 62, 63), the electrode (32, 33, 62, 63) and/or the electrode carrier (31, 61a-c) has/have a cavity (64, 65) and the temperature control device (35, 64, 65, 84) contains a time-controlled heating device for controlling the temperature of the fluid of a fluid source (83, 85) which is connected via a throughflow quantity controller (84, 87) to the electrode (32, 33, 62, 63) and/or the electrode carrier (31, 61a-c), and an effective temperature profile control device (87) is provided which is connected via a control signal connection to the temperature control device (35, 64, 65, 84).

2. Electrosurgery apparatus according to claim 1, **characterised in that** the electrode (32, 33, 62, 63) and the electrode carrier (31, 61a-c) can be heated to a temperature of more than 30°.

3. Electrosurgery apparatus according to claim 2, **characterised in that** the electrode (32, 33, 62, 63) and the electrode carrier (31, 61a-c) can be heated to a temperature of more than 37°.

4. Electrosurgery apparatus according to any of claims 1 to 3, **characterised in that** the electrode (32, 33, 62, 63) and/or the electrode carrier (31, 61a-c) has/have a thermoelectric heating and cooling device.

5. Electrosurgery apparatus according to any of the preceding claims, **characterised in that** for controlling the alternating current power, the effective temperature profile control device (87) is connected to the alternating current source (81) via a control input.

6. Electrosurgery apparatus according to claim 5, **characterised in that** the effective temperature profile control device (87) comprises an interactively programmable calculation unit (87.2) for determining simulated, time-dependent effective temperature profiles on the basis of parameters of the tissue and the electrode (82) and assumed parameters of the alternating current source (81) and the temperature control device (83), and for varying the assumed parameters to ascertain an optimised, time-dependent effective temperature profile.

7. Electrosurgery apparatus according to either claim 5 or 6, **characterised by** at least one low-inertia temperature sensor (86) connected to an input of the effective temperature profile control device (87) and which can be arranged in the body adjacent to the electrode (32, 33, 62, 63) or the electrode carrier (31, 61a-c).

8. Electrosurgery apparatus according to claim 7, **characterised in that** the temperature sensor (86) is connected to an input of the calculation unit (87.2) and that the calculation unit (87.2) comprises means for verification or correction of a simulated, time-dependent effective temperature profile on the basis of the measurement signal of the temperature sensor.

9. Electrosurgery apparatus according to either claim 5 or 6, **characterised by** impedance measuring means (86) constructed on the electrode carrier (31; 61a, 61b) for measuring the impedance between two electrodes (32, 33; 62, 63) and connected to an input of the effective temperature profile control device (87).

10. Electrosurgery apparatus according to claim 9, **characterised in that** for controlling an HF output power to be emitted via the electrodes (32, 33, 62, 63), the impedance measuring means (86) are connected to the alternating current source (81).

11. Electrosurgery apparatus according to claim 9, **characterised in that** for controlling the heating and cooling power by a fluid pump (84) and/or a fluid heater (85) as a function of an impedance measurement, the effective temperature profile control device (87) is formed by the impedance means (86).

12. Electrosurgery apparatus according to any of the preceding claims, **characterised in that** the effective temperature profile control device comprises means (87.1, 87.2, 87.6) for storing and retrieving the time dependency of control signals and for emitting control signals according to a stored time dependency.

13. Electrosurgery apparatus according to any of the preceding claims, **characterised in that** the electrode carrier (31, 61a-c) comprises a tubular element (61a, 61b) made of an electrically insulating material with a tapering, in particular conical distal end, on the circumferential surface of which the electrode (62, 63) is arranged and in the interior of which the temperature control device (64, 65) is arranged.

14. Electrosurgery apparatus according to any of the preceding claims, **characterised by** two electrodes (32, 33; 62, 63) on the electrode carrier (31; 61a, 61b).

15. Electrosurgery apparatus according to any of the preceding claims, **characterised in that** a temperature control fluid is sterile, distilled water.

## Revendications

1. Dispositif d'électrochirurgie équipé d'un porte-électrodes (31,61a-c), d'au moins une électrode (32,33,62,63) sur le porte-électrodes (31,61a-c), d'une source de courant alternatif (81) reliée de façon électriquement conductrice par l'intermédiaire d'un câble à l'électrode (32,33,62,63) et d'un dispositif de modération de température (35, 64, 65, 84) pour l'électrode (32,33,62, 63) et le porte-électrodes (31, 61a-c), le porte-électrodes (31, 61a-c) présentant une pointe à son extrémité distale en vue de l'introduction directe dans un tissu corporel, et des moyens (85) étant prévus à l'aide desquels l'électrode (32,33,62,63) et le porte-électrodes (31, 61a-c) peuvent être chauffés indépendamment de l'intensité du courant électrique à travers l'électrode (32,33,62,63), l'électrode (32,33,62,63) et/ou le porte-électrodes (31,61a-c) présentant une cavité (64,65) et le dispositif de modération de température (35,64,65,84) contenant un organe de chauffage pouvant être commandé temporellement en vue de la modération de la température du fluide d'une source de fluide (83,85), qui est en communication par l'intermédiaire d'un dispositif de commande de débit (84,87) avec l'électrode (32,33,62,63) et/ou le porte-électrodes (31,61a-c) et un dispositif de commande du profil de température effective (87) est prévu, qui est relié par l'intermédiaire d'une liaison de signaux de commande au dispositif de modération de température (35,64,65,84).

2. Dispositif d'électrochirurgie selon la revendication 1, **caractérisé en ce que** l'électrode (32,33,62,63) et le porte-électrodes (31,61a-c) peuvent être chauffés à une température supérieure à 30°.

3. Dispositif d'électrochirugie selon la revendication 2, **caractérisé en ce que** l'électrode (32,33,62,63) et le porte-électrodes (31,61a-c) peuvent être chauffés à une température supérieure à 37°.

4. Dispositif d'électrochirurgie selon une des revendications 1 à 3, **caractérisé en ce que** l'électrode (32,33,62,63) etLou le porte-électrodes (31,61a-c) présente un dispositif de chauffage et de refroidissement thermoélectrique.

5. Dispositif d'électrochirurgie selon une des revendications précédentes, **caractérisé en ce que** le dispositif de commande du profil de température effective (87) est relié en vue de la commande de la puissance du courant alternatif par l'intermédiaire d'une entrée de commande à la source de courant alternatif (81).

6. Dispositif d'électrochirurgie selon la revendication 5, **caractérisé en ce que** le dispositif de commande du profil de température effective (87) comprend une unité de calcul programmable interactive (87.2) en vue de la détermination de profils de température effective en fonction du temps simulés à partir de paramètres du tissu et de l'électrode (82) et de paramètres supposés de la source de courant alternatif (81) et du dispositif de modération de température (83), et en vue de la variation des paramètres supposés en vue de la détection d'un profil de température effective en fonction du temps optimisé.

7. Dispositif d'électrochirurgie selon une des revendications 5 ou 6, **caractérisé par** au moins un capteur de température à faible inertie (86), qui est relié à une entrée du dispositif de commande du profil de température effective (87) et peut être disposé dans le corps adjacent à l'électrode (32,33,62,63) ou au porte-électrodes (31,61a-c).

8. Dispositif d'électrochirurgie selon la revendication 7, **caractérisé en ce que** le capteur de température (86) est relié à une entrée de l'unité de calcul (87.2) et **en ce que** l'unité de calcul (87.2) présente des moyens en vue de la vérification ou de la correction d'un profil de température effective en fonction du temps, simulé à partir du signal de mesure du capteur de température.

9. Dispositif d'électrochirurgie selon une des revendications 5 ou 6, **caractérisé par** des moyens de mesure d'impédance (86), qui sont réalisés sur le porte-électrodes (31;61a,61b) en vue de la mesure de l'impédance entre deux électrodes (32,33;62,63) et sont reliés à une entrée du dispositif de commande du profil de température effective (87).

10. Dispositif d'électrochirurgie selon la revendication 9, **caractérisé en ce que** les moyens de mesure d'impédance (86) sont reliés à la source de courant alternatif (81) en vue de la commande d'une puissance de sortie-HF à délivrer par l'intermédiaire des électrodes (32, 33; 62, 63).

11. Dispositif d'électrochirurgie selon la revendication 9, **caractérisé en ce que** le dispositif de commande du profil de température effective (87) en vue de la commande de la puissance de chauffage et de refroidissement est réalisé par une pompe à fluide (84) et/ou un organe de chauffage de fluide (85) en fonction d'une mesure d'impédance par les moyens de mesure d'impédance (86).

12. Dispositif d'électrochirurgie selon une des revendications précédentes, **caractérisé en ce que** le dispositif de commande du profil de température effective présente des moyens (87.1,87.2,87.6) en vue de la mémorisation et de la délivrance de la fonction du temps de signaux de commande et en vue de la délivrance de signaux de commande correspondant à une fonction du temps mémorisée.

13. Dispositif d'électrochirurgie selon une des revendications précédentes, **caractérisé en ce que** le porte-électrodes (31,61a-c) présente un élément tubulaire (61a,61b) en matériau électriquement isolant avec une extrémité distale s'amincissant, en particulier conique, sur la surface latérale duquel est disposée l'électrode (62,63) et dans l'intérieur duquel est disposé le dispositif de modération de température (64,65).

14. Dispositif d'électrochirurgie selon une des revendications précédentes, **caractérisé par** deux électrodes (32,33;62,63) sur le porte-électrodes (31;61a,61b).

15. Dispositif d'électrochirurgie selon une des revendications précédentes, **caractérisé en ce que** le fluide de modération de température est de l'eau distillée stérile.
